Europäisches Patentamt

European Patent Office

Office européen des brevets

(11) Veröffentlichungsnummer: **0 455 014 A1**

# EUROPÄISCHE PATENTANMELDUNG

(21) Anmeldenummer: 91105716.4

(22) Anmeldetag: 11.04.91

(51) Int. Cl.5: **D01H 13/32**

(30) Priorität: 27.04.90 CH 439/90
27.04.90 CH 440/90

(43) Veröffentlichungstag der Anmeldung:
**06.11.91 Patentblatt 91/45**

(84) Benannte Vertragsstaaten:
**CH DE FR GB IT LI**

(71) Anmelder: **MASCHINENFABRIK RIETER AG**

**CH-8406 Winterthur(CH)**

(72) Erfinder: **Wicki, Raphael**
**Sonnenhof 2**
**CH-8355 Aadorf(CH)**

(54) Vorrichtung zur Messung der Masse eines Faserbandes.

(57) In einer Spinnerei ist die Masse eines Faserbandes für die Herstellung einwandfreier Vorgarne zu vergleichmässigen.

Die Vergleichmässigung erfolgt durch eine Messung der Dicke des Faserbandes, nachdem es in einem Spalt zwischen zwei Walzen auf ein bestimmtes Volumen verdichtet wurde. Eine Walze ist starr angeordnet während die andere mit ihr federnd in Eingriff steht. Auslenkungen der federnd gelagerten Walze sind ein Mass für Dickenabweichungen, und die Auslenkungen werden durch einen Sensor (44) aufgenommen, der Einrichtungen zur Vergleichmässigung des Masseanfalles steuert.

Bekannte Einrichtungen zu diesem Zweck weisen ein Eigenschwingungsverhalten auf, das die Messwerte der Messwalzen bei bestimmten Betriebszuständen verfälscht. Die erfindungsgemässe Messeinrichtung (10) ist konstruktiv so ausgestaltet, dass ihr Eigenschwingungsverhalten so ist, dass sie bei beliebigen Betriebszuständen genaue Messwerte liefert. Im übrigen ist die Messeinrichtung (10) leicht zu beschicken.

Fig.1

EP 0 455 014 A1

Die Erfindung betrifft eine Vorrichtung zur kontinuierlichen

Messung der Masse eines laufend geförderten Faserbandes gemäss Oberbegriff des ersten Anspruches.

Im Spinnprozess obliegt dem Streckwerk z.B. einer Strecke die Aufgabe, ein Faserband zu vergleichsmässigen bevor aus einem so aufbereiteten Faserband Vorgarn erzeugt wird. Die Vergleichsmässigung erfolgt durch die Doublierung mehrerer Faserbänder und durch Veränderung bzw. Regelung der Drehzahlen von Bändern fördernden Walzen in einem Verzugsbereich des Streckwerks einer Strecke oder durch Drehzahländerungen von Speisewalzen am Eingang einer Faserbänder erzeugenden Karde. Ueber- oder unterschreitet die Masse eines laufend geförderten Faserbandes eine vorbestimmte Grössenordnung, so werden die Abweichungen durch Drehzahländerungen zum Zwekke des vergleichmässigenden Mehr- oder Minderverzuges ausgeglichen. Die Drehzahländerungen werden durch eine Messeinrichtung und eine damit zusammenwirkende Steuereinrichtung für Drehzahländerungen der Walzenantriebe vorgenommen. Dazu wird das Faserband in einer Messeinrichtung auf ein bestimmtes Mass komprimiert und die Dikke des so gebildeten Volumens gemessen, wobei Abweichungen von einer vorbestimmten, die Bandvergleichmässigung identifizierenden Dicke Signale für die Steuereinrichtungen auslösen.

Bekannt ist eine Messeinrichtung vorerwähnter Wirkungsweise, die bezüglich ihrer Achslagerungen aus einer ortsfesten Treibwalze und einer bewegbaren Schleppwalze besteht, die zwischen sich einen in seiner Dimension veränderbaren Walzenspalt bilden. Die angetriebene Treib- und die über Reibung mitgenommene Schleppwalze sind als Stufenwalzen ausgebildet, die so miteinander in Eingriff stehen, dass ein in Durchlaufrichtung eines Faserbandes offener ansonsten aber durch Walzenumfänge und Stufen allseits begrenzter Walzenspalt entsteht, in dem bei Durchlauf das Faserband zur Dickenmessung komprimiert wird. Die Achslagerungen der Schleppwalze sind mit Blattfedern an einer Verankerung so vorgenommen, dass die Achslagerungen Pendelbewegungen ausführen können. Weiter wirken auf die Achslagerungen der Schleppwalze Federn ein, deren Kräfte so eingestellt sind, dass die Schleppwalze mit einem bestimmten Druck an die Treibwalze gedrückt wird. Bei Masseanfall bedingter Veränderung des komprimierten Bandvolumens erweitert oder schliesst sich der Walzenspalt unter Pendelverstellung der Achslage der Schleppwalze, wobei das Mass der Pendelverstellung von einem Sensor zur Auslösung von Steuersignalen aufgenomen wird.

Die bekannte Vorrichtung stellt ein Schwingungsgebilde dar, bei dem sich im Betrieb abklingend gedämpfte Eigenschwingungen überlagern. Bedingt durch die konstruktive Ausgestaltung - Trägheitskräfte ihrer schwingenden Massen, Richtkräfte und dämpfende Widerstände ihrer Bauteile sowie Wirkungsweise der durch die Schleppwalze ausgelösten äusseren Erregerkräfte - verfällt die Vorrichtung bei besonderen Betriebszuständen in ein Schwingungsverhalten mit Eigenfrequenzen, die Messergebnisse der Vorrichtung verfälschen, was zu einem nicht ausreichend vergleichmässigtem Faserband führt. Ferner ist der neue Faserbandanfang bei der bekannten Vorrichtung nur unter beträchtlichem Aufwand zwischen Treib- und Schleppwalze einbringbar.

Hiervon ausgehend hat sich der Erfinder die Aufgabe gestellt, eine Vorrichtung mit aus vorstehend beschriebenem Stand der Technik bekannten Messwalzen einschliesslich ihrer Wirkungsweise zu schaffen, die Vorrichtung nebst einer Erleichterung bzw. Vereinfachung der Bandeinfuhr jedoch konstruktiv so auszugestalten, dass ihr Schwingungsverhalten in allen Betriebszuständen Eigenschwingungen nur in solchen Frequenzbereichen zur Entstehung kommen lässt, die die Messergebnisse der Messwalzen nicht verfälschen und die Aufgabe wird erfindungsgemäss durch die im Kennzeichen des ersten Anspruches aufgeführten Merkmale gelöst.

Durch die erfindungsgemässe Ausgestaltung weist die Messeinrichtung im Betrieb Eigenschwingungen in Frequenzbereichen auf, die die Messergebnisse nicht mehr verfälschen.

Vorteilhafte Ausgestaltungen sind in den Unteransprüchen aufgeführt.

Weitere Vorteile, Merkmale und Einzelheiten der Erfindung ergeben sich aus der nachfolgenden Beschreibung bevorzugter Ausführungsbeispiele und der Zeichnung. Es zeigt:

Fig. 1:    eine Ausführungsform der erfindungsgemässen Vorrichtung in der Draufsicht, teilweise im Schnitt;

Fig. 2:    eine Seitenansicht der Vorrichtung gemäss Fig. 1;

Fig. 3:    eine Teilansicht des Walzenpaares gemäss Fig. 1 in der Draufsicht, wobei eine Walze im Schnitt dargestellt ist;

Fig. 4:    eine Teilansicht eines Walzenpaares in einer weiteren Ausführungsform in der Draufsicht, wobei eine Walze im Schnitt dargestellt ist.

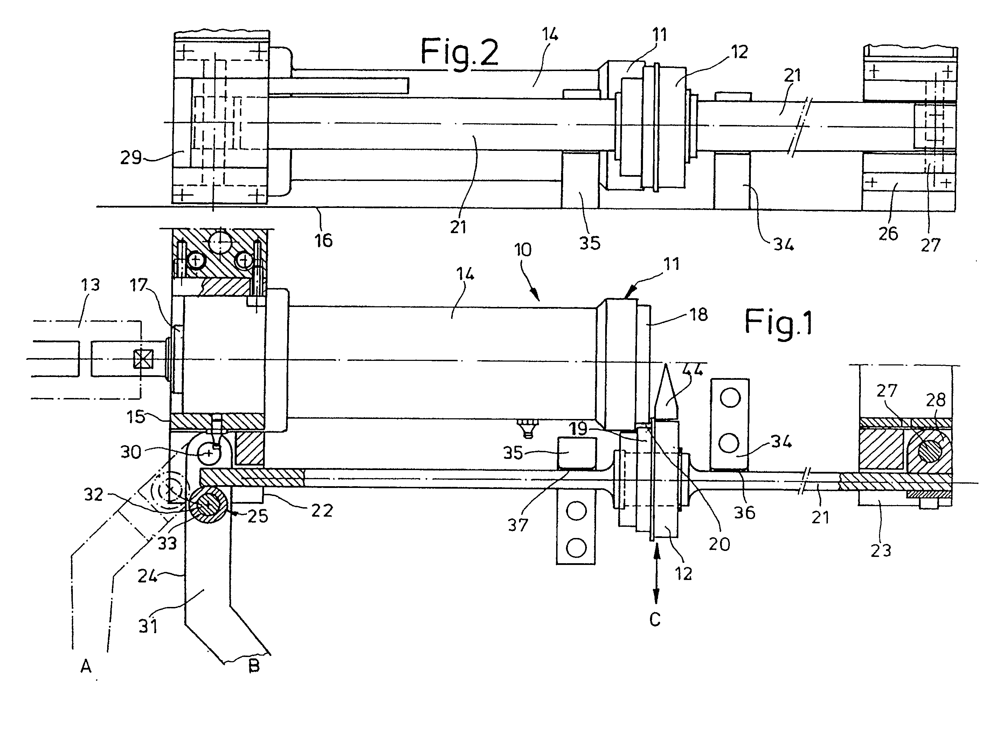

Gemäss Figur 1 besteht die erfindungsgemässe Messeinrichtung 10 aus einem Walzenpaar, das von einer Treibwalze 11 und Schleppwalze 12 gebildet ist. Die Treibwalze 11 wird durch einen Antrieb 13 angetrieben, wobei die Schleppwalze 12 durch Reibung von der Treibwalze 11 in Drehung versetzt wird.

Die Antriebswelle 17 der Treibwalze 11 ist in einer zylindrischen Hülse 14 in bekannter Weise gelagert, die ihrerseits einseitig in einem Lagerbock 15 aufgenommen ist, der mit der Grundplatte 16 in Eingriff steht. Die Treibwalze 11 ist durch entsprechende Lagerungen ihrer Antriebswelle 17 in der Hülse 14 in Radial - wie Längsrichtung spielfrei gelagert.

Die Ausbildung der Aufnahme der Hülse 14 im Lagerbock 15 gewährleistet darüber hinaus eine starre Lagerung der Hülse 14, so dass die fliegend gelagerte Treibwalze in ortsfester Achslage dreht. Die ortsfeste Lage der Drehachse der Treibwalze 11 ist für die Funktionsweise der Messeinheit insofern von Bedeutung, als ein Umfangteil der Treibwalze 11 als feststehender Messbezugspunkt zu dienen hat.

Die Treibwalze 11 und die Schleppwalze 12 sind als sogenannte Stufenwalzen ausgebildet. Die Stufe 18 der Treibwalze 11 steht mit der Stufe 19 der Schleppwalze 12 so in Eingriff, dass ein in Durchlaufrichtung des Faserbandes offener, sonst aber ein allseitig geschlossener Walzenspalt 20 in Form eines in horizontaler Lage allseits abgegrenzten quadratischen oder rechteckigen Querschnittes entsteht.

Die Schleppwalze 12 ist mittig an einem Träger 21 angeordnet, der beidseits in Lagerungen 22, 23 gehalten ist, wobei die Lagerungen 22, 23 mit der Grundplatte 16 verbunden sind. Der Träger 21 ist im Querschnitt rechteckig ausgebildet und so angeordnet, dass die längere Querschnittsseite senkrecht steht und die kürzere Seite waagerecht liegt. Die Bemessung des Querschnittes ist so getroffen, dass der Träger 21 in senkrechter Richtung ein sehr viel grösseres Trägheitsmoment aufweist als in waagerechter Richtung, so dass der Träger 21 unter Belastungseinwirkung nur in horizontaler, nicht aber in senkrechter Richtung, durchbiegt.

Diese Bemessung des Trägers 21 ist bevorzugt, wenn die Schleppwalze nicht zu einem federnd exzentrischen Umlauf um ihre Drehachse ausgebildet ist. Im Falle der Ausbildung der Schleppwalze zu einem exzentrischen Umlauf um ihre Drehachse kann der Träger 21 eine beliebige Querschnittsform aufweisen, wobei der Träger 21 so zu bemessen ist, dass er in einem durch die Versetzung der Laufhülse 39 relativ zu ihrer Drehlagerung 40 ausgelösten, mittig wirkenden Kraftangriff nicht durchbiegt, d.h. im Rahmen der auf ihn einwirkenden Kräfte als starr anzusehen ist.

Der Träger 21 kann aus metallischen und nichtmetallischen Werkstoffen gefertigt sein. Als metallischer Werkstoff kommt Stahl als nichtmetallischer Werkstoff ein Kunststoff, vorzugsweise faserverstärkter Kunststoff, in Betracht. Träger 21 können auch aus einer Schichtung von metallischen oder nichtmetallischen Trägerelementen gebildet sein. Auch kann es zweckmässig sein, einen Träger 21 mittels einer Schichtung aus metallischen, und nichtmetallischen Trägerelementen aufzubauen. Aus Schichtungen gebildete Träger 21 haben den Vorteil, vermittels unterschiedlicher Elementmaterialien auf das Dämpfungsverhalten eines Trägers 21 in bestimmter Weise Einfluss nehmen zu können.

Die Lagerung 23 des Trägers 21 ist als Schwenklager, die Lagerung 22 als eine Hebeleinrichtung 24 mit Widerlager 25 ausgebildet. Das Lager 23 umfasst einen senkrecht zur Grundplatte 16 in einem Lagerblock 26 gehaltenen zylindrischen Stift 27 und einen darum drehbaren Scharnierkörper 28. Der Träger 21 ist mit dem Scharnierkörper 28 verbunden und schwenkt so um den Stift 27.

Das Lager 22 besteht aus einem mit der Grundplatte 16 verbundenen Lagerblock 29, der einen senkrecht verlaufenden runden Drehstift 30 aufweist, um den die Hebeleinrichtung 24 dreht. Die Hebeleinrichtung 24 umfasst einen Hebelarm 31, der auf seiner der Grundplatte 16 zugewandten Seite ein Widerlager 25 trägt, das aus einer um einen Stift 32 drehbaren Hülse 33 gebildet ist.

Fig. 1 zeigt den Hebelarm 31 in zwei Schwenkstellungen "A" und "B". In Schwenkstellung "A" kann der Träger 21 um den Stift 27 ausgeschwenkt werden, während in Schwenkstellung "B" die Längsachse des Trägers 21 parallel zur Drehachse der Treibwalze 11 ausgerichtet gehalten ist. Beidseits der Schleppwalze 12 ist je ein Endanschlag 34 und 35 auf der Grundplatte 16 angeordnet, an deren Anschlagflächen 36 und 37 die der Treibwalze 11 zugewandte Seite des Trägers 21 zur Anlage kommt, während die Hülse 33 des Widerlagers 25 auf der dieser gegenüberliegenden Seite in Schliesstellung der Hebeleinrichtung 24 zur Anlage kommt. Der Träger 21 ist somit in Bewegungsrichtung "C" im Falle einer konzentrisch umlaufenden Laufhülle 39 durchbiegbar, bei exzentrisch umlaufender Laufhülse 39 nicht durchbiegbar gelagert.

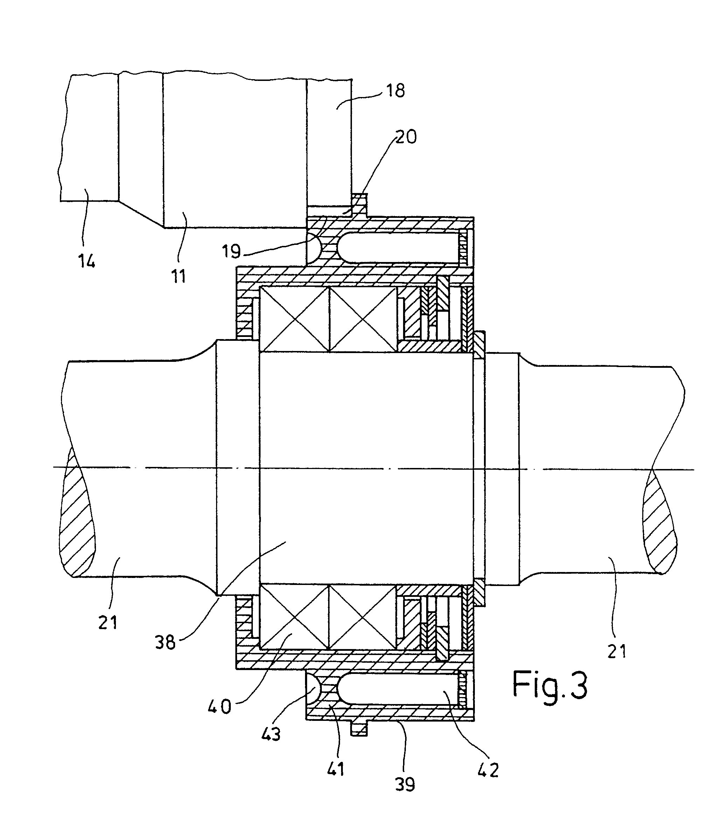

Fig. 3 zeigt einen Teil der Treibwalze 11 und zeigt die Schleppwalze 12 im Schnitt. Die Schleppwalze 12 ist auf einem runden Wellenstück 38, das Teil des ansonsten rechteckigen Trägers 21 ist, gelagert. Die Schleppwalze 12 ist aus einer Laufhülse 39 gebildet, die vermittels einer Drehlagerung 40 z.B. in Form von Spindellagern um das Wellenstück 38 dreht. Die Laufhülse 39 ist auf der Drehlagerung so angebracht, dass sie in Achsrichtung des Wellenstückes 38 keine Bewegungen ausführen kann.

Damit die Laufhülse 39 eine möglichst kleine bewegte Masse aufweist, sind im ringförmigen Mantel 41 der Laufhülse 39 sich in Achsrichtung erstreckende Ausnehmungen 42, 43 vorgesehen.

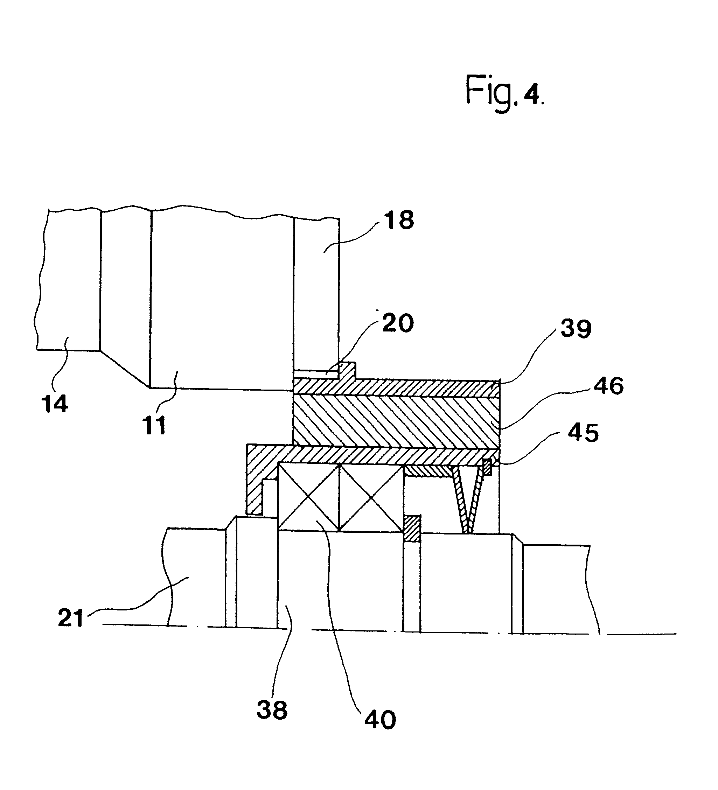

Fig. 4 zeigt einen Teil der Treibwalze 11 und eine weitere erfindungsgemäss ausgebildete Schleppwalze 12 im Schnitt. Die Schleppwalze 12 ist auch auf einem runden Wellenstück 38, das Teil des Trägers 21 ist, gelagert. Die Schleppwalze 12 ist aus einer Laufhülse 39 und einem Innenring 45 gebildet, die vermittels einer Drehlagerung 40 z.B. in Form von Spindellagern um das Wellenstück 38 drehen. Laufhülse 39 und der mit der Drehlagerung 40 in Eingriff stehende Innenring 45 sind auf der Drehlagerung 40 so angebracht, dass sie in Achsrichtung des Wellenstückes 38 keine Bewegungen ausführen können. Zwischen der Laufhülse 39 und dem Innenring 45, die aus einem metallischen Werkstoff, beispielsweise Stahl gefertigt sind, befindet sich eine Zwischenschicht 46 aus einem nichtmetallischen, elastischen Werkstoff, vorzugsweise aus Gummi oder einem Silikonkautschuk. Gummi oder Silikonkautschuk sind aufgrund ihrer hohen Eigendämpfung als federnde Werkstoffe für die Zwischenschicht bevorzugt. Zweck der Zwischenschicht 46 ist zum einen in Drehrichtung der Schleppwalze 12 zwischen Laufhülse 39 und Innenring 45 eine schlupffreie Verbindung herzustellen, zum anderen einen in radialer Richtung zwischen Laufhülse 39 und Innenring 45 versetzten, d.h. exzentrischen Umlauf der Laufhülse 39 um den Innenring 45 zur Erzielung der erfindungsgemässen Wirkungsweise der Schleppwalze 12 zu erreichen, wobei die hohe Eigendämpfung des Werkstoffes der Zwischenschicht 46 nur Schwingungen der Vorrichtung in Frequenzbereichen zulässt, die sich nicht nachteilig auf die durch Treibwalze 11 und Schleppwalze 12 ermittelten Messwerte auswirken.

Die Betriebsweisen der erfindungsgemässen Messeinrichtungen sind folgende:

Durch Bewegung der Hebeleinrichtung 24 in Schwenklage "A" wird der Träger 21 entriegelt und kann um den zylindrischen Stift 27 geschwenkt werden, so dass durch Fortbewegen der Schleppwalze 12 von der Treibwalze 11 der Walzenspalt 20 geöffnet wird. Nach Einlegen eines Faserbandes in den offenen Walzenspalt 20 wird letzterer durch Bewegung der Hebeleinrichtung 24 in Schwenklage "B geschlossen. Dabei wird das Faserband unter Auspressen im Faserband enthaltener Luft auf ein Volumen entsprechend der Dimensionierung des Walzenspaltes komprimiert. Die Treibwalze 11 wird über den Antrieb 13 in Drehung versetzt und treibt über die Reibung die Schleppwalze 12 an, so dass das Faserband unter fortlaufender Verdichtung durch den Walzenspalt 20 gefördert wird.

Während der Förderung tastet die Schleppwalze 12 Veränderungen des Masseanfalles von Faserband auf dem Wege der Dickenmessung des komprimierten Volumens im Walzenspalt 20 ab. Veränderungen in der Dicke, beispielsweise eine Dickenzunahme, bewirkt ein Duchbiegen des Trägers 21 in Bewegungsrichtung "C", (Schleppwalzenausführung nach Fig. 3) oder ein exzentrisches Auslenken der Laufhülse 39 (Schleppwalzenausführung nach Fig. 4), wodurch der Walzenspalt 20 durch Abrücken der Schleppwalze 12 von der Treibwalze 11 geöffnet wird. Die der Durchbiegung des Trägers 21 entsprechende Lageveränderung der Schleppwalze 12 bzw. deren Lageänderung durch exzentrische Auslenkung der Laufhülse 39 wird an ihrem Umfang durch einen Sensor 44, vorzugsweise auf dem Wege der berührungslosen Abstandsmessung, aufgenommen, der über an sich bekannte Einrichtungen Signale für Drehzahländerungen von Förderwalzen in der Strecke oder der Speisewalzen einer Karde auslöst.

Die erfindungsgemässen Messeinrichtungen 10 wurden vorstehend im Zusammenhang mit einer Karde oder Strecke beschrieben, um Faserband zu vergleichmässigen. Darauf sind ihre Anwendungen jedoch nicht beschränkt, sie eignen sich auch zur Verwendung mit anderen Textilmaschinen, bei denen Band- oder Garnmessungen vorzunehmen sind.

**Patentansprüche**

1. Vorrichtung zur kontinuierlichen Messung der Masse eines laufend geförderten Faserbandes, bestehend aus einer angetriebenen Treibwalze und einer über die Treibwalze angetriebenen Schleppwalze, mit jeweils stufenförmig ausgebildeten zwischen Treibwalze und Schleppwalze einen allseits begrenzten Walzenspalt bildenden Umfangsflächen, wobei die

   Achse der Treibwalze ortsfest und die Achse der Schleppwalze unter Federvorspannung relativ zur Achse der Treibwalze bewegbar angeordnet sind und einem Auslenkungen der Schleppwalze aufnehmenden Sensor,

   dadurch gekennzeichnet,

   dass die Schleppwalze (12) mittels eines beidseits in Lagerungen (22, 23) lösbar eingespannten Trägers (21) gelagert ist.

2. Vorrichtung nach Anspruch 1, dadurch gekennzeichnet, dass der Träger (21) im Querschnitt rechteckig ausgebildet ist und in Richtung seiner senkrechten Querschnittsfläche ein grösseres Trägheitsmoment aufweist, als in Richtung seiner waagrechten Querschnittsfläche.

3. Vorrichtung nach Anspruch 1 oder 2, dadurch gekennzeichnet, dass die Schleppwalze (12) mittig zwischen den Lagerungen (22, 23) des Trägers (21) angeordnet ist.

4. Vorrichtung nach einem der Ansprüche 1 bis 3, dadurch gekennzeichnet, dass der Träger (21) aus einer Mehrzahl im Querschnitt rechteckiger metallischer Trägerelemente gebildet ist.

5. Vorrichtung nach einem der Ansprüche 1 bis 3, dadurch gekennzeichnet, dass der Träger (21) aus Kunststoff, vorzugsweise einem faserverstärkten Kunststoff gebildet ist.

6. Vorrichtung nach einem der Ansprüche 1 bis 3, dadurch gekennzeichnet, dass der Träger (21) aus einer Schichtung von metallischen und nichtmetallischen, vorzugsweise aus faserverstärktem Kunststoff gebildeten Trägerelementen besteht.

7. Vorrichtung nach einem der Ansprüche 1 bis 6, dadurch gekennzeichnet, dass die Lagerungen (22, 23) des Trägers (21) an einem Ende aus einem den Träger haltenden, aus einem Stift (27) und Scharnierkörper (28) gebildeten Schwenklager und am anderen Ende aus einer schwenkbaren Hebeleinrichtung (24) mit einem Widerlager (25) besteht.

8. Vorrichtung nach einem der Ansprüche 1 bis 7, dadurch gekennzeichnet, dass die Drehachse des Schwenklagers (27, 28) parallel zu einer Senkrechten durch den Walzenspalt (20) verläuft.

9. Vorrichtung nach einem der Ansprüche 1 bis 8, dadurch gekennzeichnet, dass beidseits der Schleppwalze (12) mit der Treibwalze (11) zugewandten Seite des Trägers (21) in Eingriff bringbare Endanschläge (34, 35) vorgesehen sind.

10. Vorrichtung nach einem der Ansprüche 1 bis 9, dadurch gekennzeichnet, dass die Schleppwalze (12) eine mittels einer Drehlagerung (40) auf einem Wellenstück (38) drehbar gelagerte Laufhülse (39) kleiner Masse aufweist.

11. Vorrichtung nach einem der Ansprüche 1 bis 10, dadurch gekennzeichnet, dass die Laufhülse (39) in ihrem Mantel (41) in Achsrichtung der Laufhülse (39) verlaufende Ausnehmungen (42, 43) aufweist.

12. Vorrichtung nach einem der Ansprüche 1 bis 11, dadurch gekennzeichnet, dass in die Ausnehmung (42) im Mantel (41) der Laufhülse (39) ein schwingungsdämpfender Werkstoff, vorzugsweise ein Kunststoff oder Gummi, eingebracht ist.

13. Vorrichtung nach einem der Ansprüche 1 bis 9, dadurch gekennzeichnet, dass die Schleppwalze (12) zu einem federnd exzentrischen Umlauf um ihre Drehachse ausgebildet ist.

14. Vorrichtung nach Anspruch 13, dadurch gekennzeichnet, dass die Schleppwalze (12) aus einem Innenring (45) und einer Laufhülse (39) mit zwischen Innenring (45) und Laufhülse (39) angeordneten Zwischenschicht (46) aus einem federnd elastischen Werkstoff besteht, wobei der Innenring (45) über eine Drehlagerung (40) mit dem Träger (21) in Eingriff steht.

15. Vorrichtung nach Anspruch 13 oder 14, dadurch gekennzeichnet, dass der Werkstoff der Zwischenschicht (46) eine hohe Eigendämpfung aufweist.

16. Vorrichtung nach einem der Ansprüche 13 bis 15, dadurch gekennzeichnet, dass der Träger (21) in seinem Querschnitt so bemessen ist, dass er bei einem durch die Versetzung der Laufhülse (39) relativ zu ihrer Drehlagerung (40) ausgelösten Kraftangriff durchbiegungsfrei ist.

Fig.2

Fig.1

EP 0 455 014 A1

Fig.3

Fig.4.

Europäisches Patentamt

**EUROPÄISCHER RECHERCHENBERICHT**

Nummer der Anmeldung

| EINSCHLÄGIGE DOKUMENTE | | | EP 91105716.4 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| A | EP - A1 - 0 192 835 (MASCHINENFABRIK RIETER AG) * Ansprüche * -- | 1,13 | D 01 H 13/32 |
| A | DE - A1 - 3 826 861 (VEB KOMBINAT TEXTIMA) * Gesamt * -- | 1,7 | |
| A | DE - B2 - 1 944 733 (CARDING SPECIALISTS (CANADA) LTD.) * Ansprüche; Fig. * -- | 1,7 | |
| A | GB - A - 2 062 704 (ELITEX, KONCERN TEXTILNIHO STROJIRENSTRI) * Gesamt * ---- | 1,7 | |
| | | | RECHERCHIERTE SACHGEBIETE (Int. Cl.⁵) |
| | | | D 01 G D 01 H |

Der vorliegende Recherchenbericht wurde für alle Patentansprüche erstellt.

| Recherchenort | Abschlußdatum der Recherche | Prüfer |
|---|---|---|
| WIEN | 31-05-1991 | NETZER |